# EUROPEAN PATENT APPLICATION

(11) **EP 2 080 801 A1**
(43) Date of publication of application: **22.07.2009**
(21) Application number: 08100684.3
(22) Date of filing: 21.01.2008
(51) Int. Cl.: C12N 15/09, C12P 37/00, C12P 35/00

(54) **Production of non-ribosomal peptides in Saccharomyces**

(71) Applicant: Technical University of Denmark, 2800 Lyngby (DK)
(72) Inventor: Nielsen, Jens, 2920 Charlottenlund (DK); Siewers, Verena, 2100 Copenhagen (DK); Chen, Xiao, 2830 Virum (DK); Zhang, Jie, 2850 Nærum (DK)
(74) Representative: Benthin, Stig

(57) **Abstract**

Genetically modified *Saccharomyces* having the ability to synthesize non-ribosomal peptides.

## Description

### FIELD OF THE INVENTION

The present invention relates to the field of genetic engineering and to the field of industrial biotechnology. Particularly the present invention relates to genetically engineered *Saccharomyces,* and to the synthesis of non-ribosomal peptides using *Saccharomyces* as host.

### BACKGROUND OF THE INVENTION

Penicillin belongs to the class of β-lactams, which is a broad group of antibiotics that encompass some of the most widely used drugs, i.e. ampicillin, amoxicillin and cephalexin. The total world market for this group of antibiotics exceeds 20 billion US$. These efficient antibiotics are used for treatment of a wide range of infectious diseases and even 60 years after the discovery of penicillin they represent the most cost-efficient drugs on the market. Most β-lactams are being produced by chemical synthesis from penicillin, which is still produced through industrial fermentation with the filamentous fungus *Penicillium chrysogenum.* The current world-wide production of penicillin exceeds 60.000 tons and the production volume is growing by more than 10% annually (primarily due to rapid growth in China and India). Despite a key role of industrial biotechnology in these first generation processes for production of β-lactams, there are several chemical synthesis steps and the process requires the addition of toxic precursors like phenoxyacetic acid and phenylglycine.

Recently a second generation process has been developed for the production of cephalexin which relies on extending the penicillin biosynthetic pathway with a single additional enzymatic reaction. Hereby, it was made possible to produce the β-lactam adipoyl-7-ADCA directly by fermentation, and this resulted in removal of several chemical steps in the production of cephalexin. Apart from a 50% reduction in variable costs with the new process, the biotechnological process resulted in a 65% reduction of both the energy and material usage.

Both the first and the second generation processes for production of β-lactams rely on the use of the filamentous fungus *P. chrysogenum.* This fungus serves as an efficient cell factory for the production of penicillins and the productivity has been improved more than a 1,000 fold since the industrial process was initiated in the mid 1940ies. However, there are a number of drawbacks on the use of this cell factory: 1) the fermentation medium becomes highly viscous resulting in requirement for large energy inputs; 2) the fungus is unstable and it is therefore not possible to operate the process in a continuous mode; 3) there is relatively little known on the genetics and physiology of the fungus; and 4) it is difficult to perform genetic engineering in this organism.

β-lactams represent interesting model compounds for other antibiotics, e.g. the glycopeptides which are used as the last line of defense for fighting bacterial infections (e.g. vancomycin). Glycopeptides are formed in pathways that resemble the penicillin biosynthetic pathway, i.e. key enzymes in these pathways are the so-called non-ribosomal peptide synthetases (NRPSs) of which the first enzyme of the penicillin biosynthetic pathway (ACVS) is one of the most well studied. The family of NRPSs comprises a variety of multifunctional proteins with the ability to synthesize many pharmaceutically important compounds such as antibiotics, anti-fungal compounds and anticancer compounds. The NRPSs consist of single peptide chains or complexes of proteins containing between one and at least 17 modules comprising peptidyl carrier protein domains interspersed among the catalytic domains. The peptidyl carrier domains are post-translationally activated by phosphopantetheinyl transferases that attach a 4'-phosphopantetheine prosthetic group comprising a free thiol.

The yeast *Saccharomyces cerevisiae* is a well characterised organism widely used in the food and pharmaceutical industry due to a) being generally recognized as safe (GRAS status), b) being amenable to recombinant DNA technology and c) the whole genome has been sequenced. *Saccharomyces cerevisiae* is devoid of NRPS genes but encompasses phosphopantetheinyl transferase activity in connection with fatty acid biosynthesis, lysine biosynthesis and activation of mitochondrial acyl-carrier proteins.

WO 2006/060839 discloses a method for the identification of inhibitors of phosphopanteteinyl transferases of the primary and secondary metabolism. Also disclosed is a *Saccharomyces cerevisiae* comprising a vector comprising a gene encoding a phosphopantetheinyl transferase from *Fusarium graminearum.*

### SUMMARY OF THE INVENTION

The present invention is to provide an isolated *Saccharomyces cerevisiae* comprising at least one heterologous gene encoding a non-ribosomal protein synthetase (NRPS) catalyzing the synthesis of at least one non-ribosomal peptide.

The present invention also provides the use of *Saccharomyces cerevisiae* as host cell for the industrial manufacture of a non-ribosomal peptide. In one aspect the invention provides a process for the manufacture of a non-ribosomal peptide comprising the steps of :
a) cultivating an isolated *Saccharomyces cerevisiae* according to the invention in a suitable growth medium,
b) optionally feeding of one or more precursors for said non-ribosomal peptide during said cultivation,
c) harvesting the spent cultivation medium,
d) isolating the non-ribosomal peptide from said spent cultivation medium.

In another aspect the present invention relates to the use of an isolated *Saccharomyces* according to the invention, for the generation of a library of antibiotics.

### BREIF DESCRIPTION OF THE DRAWINGS.

Figure 1. Plasmid map of pESC-npgA-pcbAB.
Figure 2. Plasmid map of pESC-pptA-pcbAB.
Figure 3. Plasmid map of pESC-sfp-pcbAB.
Figure 4. Tri-partite strategy for integration of *npgA* and *pcbAB* into the yeast genome. Direct repeats flanking *K.I. URA3* are indicated as black bars. P₁P₁₀, bi-directional GAL1/GAL10 promoter; T_{A}, ADH1 terminator; T_{C}, CYC1 terminator; 3', 3' region of integration site; 5', 5' region of integration site; 5-FOA, 5-flouroorotic acid.
Figure 5. Plasmid map of pESC-ppp.
Figure 6. Plasmid maps of domain shuffling products.
Figure 7. Plasmid maps of pESC-sfp-tycA and pESC-HIS-srf.
Figure 8. Overview of key reactions involved in current production of β-lactams using the filamentous fungus *P. chrysogenum.* Penicillin V and adipoyl-7-ADCA are major fermentation products, which are used for subsequent chemical synthesis of the key antibiotics amoxicillin, ampicillin and cephalexin (chemical synthesis steps are indicated by a dotted line).

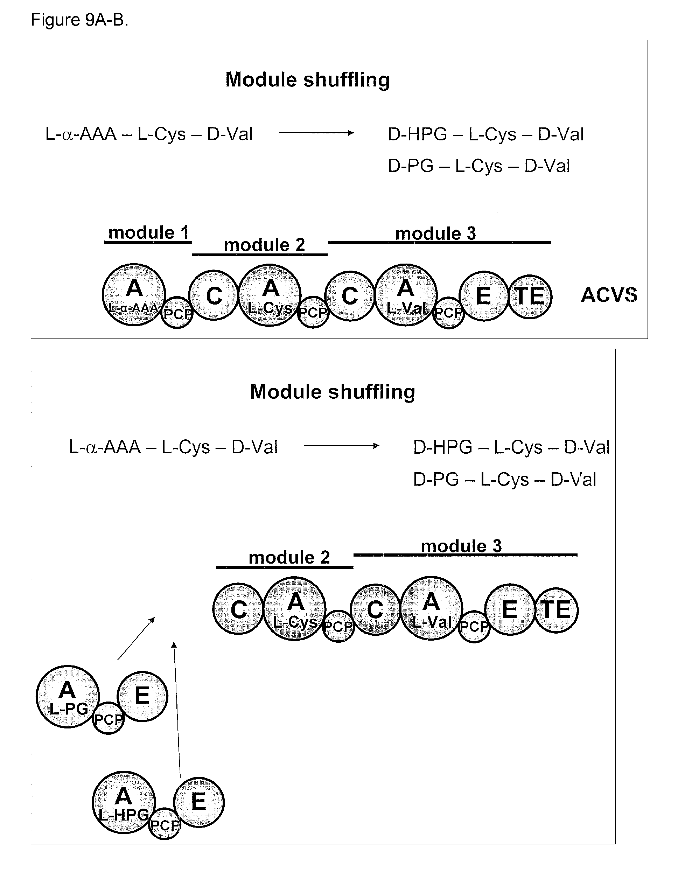

Figure 9A-B. Module shuffling.

### DETAILED DESCRIPTION OF THE INVENTION

### Biosynthetis of β-lactams in P. chrysogenum.

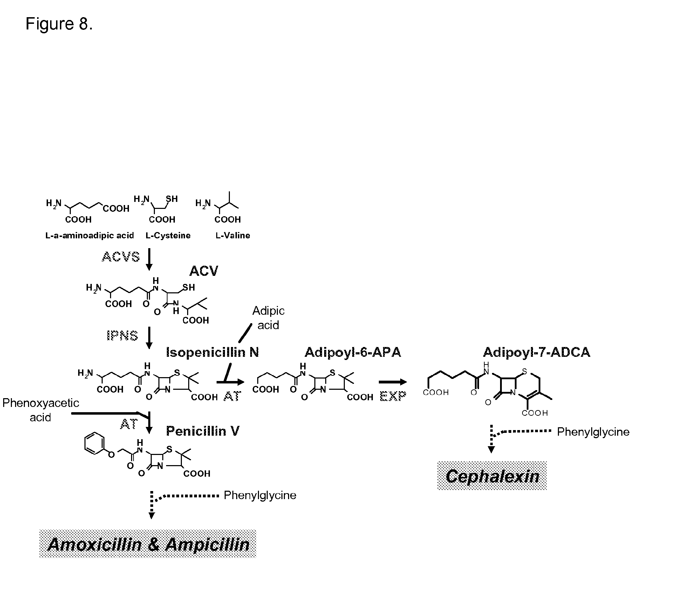

The penicillin biosynthesic pathway consists of three enzymatic steps (see figure 8). In the first step the three amino acids L-α-aminoadipate, L-cysteine and D-valine are condensed into the tripeptide δ-(L-α-aminoadipyl)-L-cysteinyl-D-valine (ACV). This reaction is catalyzed by the enzyme δ-(L-α-aminoadipyl)-L-cysteinyl-D-valine synthetase (ACVS). ACVS from various species are around 3700 amino acid residues long with molecular masses around 400 kD. ACVS comprises 3 homologous regions (modules) - one for each amino acid - where each module contains an adenylation, a peptidyl carrier and a condensation domain arranged in a characteristic order. The amino acid is recognised and activated by the respective adenylation domain, and then attached to a 4'-phosphopantetheine cofactor that is bound to the peptidyl carrier domain. Finally, the condensation domain catalyses peptide bond formation. Besides performing the activation of amino acids and formation of peptide bonds, ACVS is also responsible for forming an epimerization of valine from its L-form to its D-form.
In the second step, ACV is converted into isopenicillin N (IPN). This reaction is catalyzed by isopenicillin synthetase (IPNS), a non-heme enzyme that uses oxygen as electron acceptor.
In the last step, isopenicillin N is converted into penicillin V (or G). This reaction is catalyzed by an acyl-transferase (AT) that exchanges the α-aminoadipyl sidechain of isopenicillin N with phenoxyacetic acid (or phenylacetic acid in the case of production of penicillin G). Before exchange phenoxyacetic acid needs to be activated as a CoA-ester, and a phenylacetyl-CoA ligase is responsible for this reaction (reaction not shown in figure 8).

In the pathway towards cephalosporins (here represented as adipoyl-7-ADCA) the two first steps of the penicillin biosynthetic pathway are also used.

By feeding with adipate instead of phenoxyacetic acid the penicillin adipoyl-6-APA may be formed through action of the acyl-transferase. Adipoyl-6-APA may be expanded to form the cephalosporin adipoyl-7-ADCA by an expandase (EXP). Expandase activity is not found in *P. chrysogenum,* but in the second generation process for production of cephalexin an expandase from the bacterium *Streptomyces clavilugerus* has been expressed in this fungus. The expandase is an unusual enzyme as it requires oxygen as electron acceptor as well as it is uses 2-oxoglutarate as an additional electron acceptor.

In a first aspect the invention relates to an isolated *Saccharomyces cerevisiae* comprising at least one heterologous gene encoding a non-ribosomal protein synthetase (NRPS) catalyzing the synthesis of at least one non-ribosomal peptide.
In one embodiment the isolated *Saccharomyces cerevisiae* comprises a heterologous gene encoding 4'-phosphopantetheinyl transferase. The heterologous gene encoding 4'-phosphopantetheinyl transferase may be endogeneous to the *Saccharomyces cerevisiae* or it may be a heterologous gene. In one embodiment the heterologous gene encoding 4'-phosphopantetheinyl transferase originates from a microorganism, such as from a fungal microorganism or from a prokaryote such as *Bacillus subtilis.* In an embodiment said heterologous gene encoding 4'-phosphopantetheinyl transferase originates from *Aspergillus nidulans* or from *Penicillium chrysogenum.*

In another aspect the isolated *Saccharomyces cerevisiae* according to the invention comprises at least one heterologous gene encoding a NRPS which encodes a single polypeptide. In another aspect the isolated *Saccharomyces cerevisiae* according to the invention comprises at least one heterologous gene encoding a NRPS which encodes several associated polypeptides.

In one embodiment the isolated *Saccharomyces cerevisiae* comprises at least one heterologous gene encoding a NRPS comprises a gene encoding ACV synthetase (ACVS).
In one embodiment the isolated *Saccharomyces cerevisiae* comprises at least one heterologous gene encoding a NRPS comprises a gene encoding isopenicillin synthetase (IPNS).
In one embodiment the isolated *Saccharomyces cerevisiae* comprises at least one heterologous gene encoding a NRPS comprises a gene encoding phenylacetyl-CoA ligase and a gene encoding an acyltransferase.
In another embodiment the isolated *Saccharomyces cerevisiae* also comprises a gene encoding acyltransferase and a gene encoding expandase.

In another embodiment the NRPS modules have been shuffled so as to enable said *Saccharomyces cerevisiae* to produce δ-(L-α-aminoadipyl)-L-cysteinyl-D-valine (ACV) derivatives.

In a further aspect the present invention relates to a process for the manufacture of a non-ribosomal peptide comprising the steps of:
a) cultivating an isolated *Saccharomyces cerevisiae* according to the invention in a suitable growth medium,
b) optionally feeding of one or more precursors for said non-ribosomal peptide during said cultivation,
c) harvesting the spent cultivation medium,
d) isolating the non-ribosomal peptide or precursor thereof from said spent cultivation medium.

In another aspect the present invention relates to a process for the manufacture of ampicillin, amoxicillin, cephalexin or cefadroxil comprising the steps of :
a) cultivating an isolated *Saccharomyces cerevisiae* according to the present invention in a suitable growth medium,
b) feeding of phenylglycine or p-hydroxyphenylglycine during said cultivation,
c) harvesting spent cultivation medium,
d) isolating the ampicillin, amoxicillin, cephalexin or cefadroxil from said spent cultivation medium.

In an aspect the present invention relates to the use of *Saccharomyces cerevisiae* as host cell for the industrial manufacture of a non-ribosomal peptide. In an aspect the present invention relates to the use of *Saccharomyces cerevisiae* as host cell for the synthesis of a non-ribosomal peptide. In one embodiment said *Saccharomyces cerevisiae* comprises at least one heterologous gene encoding a NRPS. In another embodiment said *Saccharomyces cerevisiae* comprises a heterologous gene encoding 4'-phosphopantetheinyl transferase.
In another embodiment the NRPS modules in said *Saccharomyces cerevisiae* has been shuffled such that at least one ACV derivative is produced. In another embodiment said at least one ACV derivative is selected from D-HPG-L-Cys-D-Val, D-PG-L-Cys-D-Val or a mixture thereof.

In another aspect the present invention relates to 19. Use of an isolated *Saccharomyces cerevisiae* according to any of claims 1-10 for the manufacture of a compound selected from the group consisting of 6-APA, 7-ADCA, ampicillin, cephalexin, amoxicillin or cefadroxil.

In another aspect the present invention relates to the use of an isolated *Saccharomyces* according to the invention for the generation of a library of non-ribosomal peptides. By shuffling the NRPS modules libraries of non-ribosomal peptides may be generated, whose non-ribosomal peptides may comprise several peptides having activity as an antibiotic. A large number of antibiotics are known in the art and a large number of methods for quantifying the activity of antibiotics are available in the art. Preferably the libraries of non-ribosomal peptides are screened for their antibiotic activity, and those libraries exhibiting activity are separated into a smaller number of peptides, or even individual peptides, in order to establish the structure activity relationship. Once this has been done, a recombinant *Saccharomyces* can be made, which produces solely the particular non-ribosomal peptide, which has the desired antibiotic activity.

The isolated *Saccharomyces cerevisiae* according to the invention can also be used for the manufacture of a NRPS.

Vectors for expression of recombinant peptides in *Saccharomyces cerevisiae* are well known in the art. Several ready-to-use cloning vectors for *Saccharomyces cerevisiae* are also commercially available. A number of these vectors are based on the 2micron plasmid, and they come with antibiotic selection markers or with selectable markers that do not require antibiotics.

The non-ribosomal peptide may be detected using methods known in the art that are specific for the peptide. These detection methods may include use of specific antibodies, formation of an enzyme product, disappearance of an enzyme substrate, or SDS-PAGE. Procedures for determining enzyme activity are known in the art.
The resulting non-ribosomal peptide can be isolated by methods known in the art. For example, the peptide may be isolated from the nutrient medium by conventional procedures including, but not limited to, centrifugation, filtration, extraction, spray drying, evaporation, or precipitation. The isolated peptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), or extraction (see, e.g., Protein Purification, J.-C. Janson and Lars Ryden, editors, VCH Publishers, New York, 1989).

The present invention is further illustrated with reference to the following examples, which should not in any way be construed as limiting the scope of the invention as defined in the description and in the claims.

### EXAMPLES

### Example 1. Plasmid based expression of ACVS together with three different PPTases in S. cerevisiae

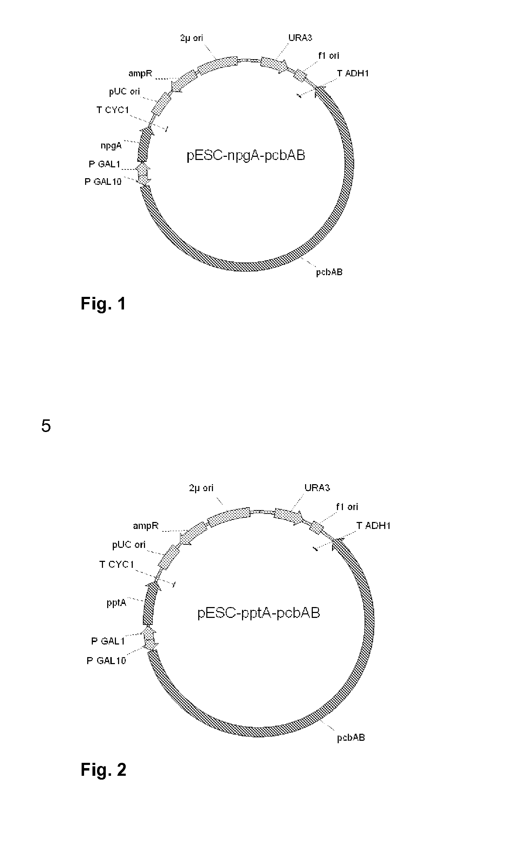

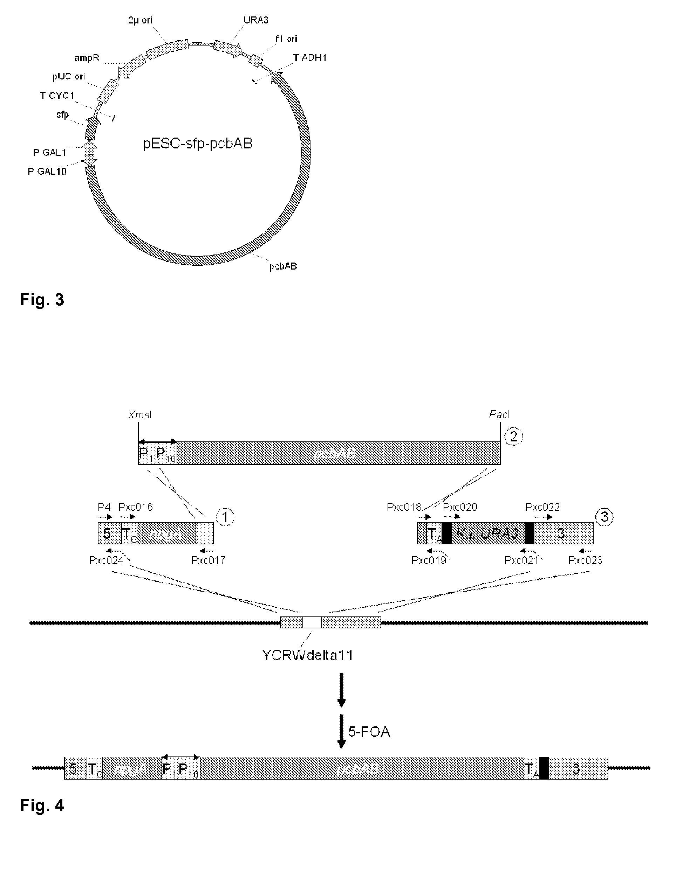

The PPTase encoding gene *npgA* from *Aspergillus nidulans* was amplified from vector pDKP4832 (kindly provided by G. Turner, University of Sheffield, UK) using primers npg-1 (5'-GGCGTCGACATGGTGCAAGACACATCAAGC-3') and npg-2 (5'-CCAAGCTTATTAGGATAGGCAATTACACAC-3'), thereby introducing a *Sa*/I and a *Hind*III restriction site (indicated in bold type), respectively. After restriction with the respective enzymes, the PCR fragment was cloned into the pESC-URA vector (Stratagene) in order to generate vector pESC-npgA. The 3' region of the ACVS encoding gene *pcbAB* from *Penicillium chrysogenum* was amplified using primers pcbAB-2 (5'-TTGTTAATTAAGTCAATAGCGAGCGAGGTGTTC-3'; containing a *Pac*I site) and pcbAB-4 (5'-TTCCTTAAGCGCTGCTGTCAG-3') as well as cosmid pCX3.2 (kindly donated by G. Turner, University of Sheffield; Smith et al. 1990) as template. The PCR product was cut with *Pac*I and *Sac*I generating a 0.9 kb fragment, which was then cloned into *Pac*I/*Sac*I restricted pESC-npgA. Subsequently, a PCR fragment containing the 5' region of *pcbAB,* which was amplified from pCX3.2 using primers pcbAB-1 (5'-AGGGCGGCCGCAATGACTCAACTG AAGCCACCG-3'; containing a *Not*I site) and pcbAB-3 (5'- AGACACGTGAAGCGATTCTCG-3') and cut with *Not*I and *Bg*/II, was cloned into this vector, which had been restricted with the same enzymes. This resulted in formation of vector pESC-npgA-5'3'. PESC-npgA-5'3' was linearised with *Bg*/II. In addition, a 12 kb *Spel* fragment containing the *pcbAB* gene was isolated from pCX3.2. Both fragments were co-transformed into S. *cerevisiae* CEN.PK113-5D (obtained from P. Kötter, University of Frankfurt, Germany), thus allowing the formation of vector pESC-npgA-pcbAB (Fig. 1) by homologous recombination. The vector was re-isolated from S. *cerevisiae* and all sequences obtained by PCR were verified by sequencing (MWG, Martinsried, Germany). pESC-npgA-pcbAB allows expression of *npgA* under control of the GAL1 promoter, while expression of *pcbAB* is controlled by the GAL10 promoter.
In order to test PPTases from other organisms than *A. nidulans* in combination with ACVS from *P. chrysogenum,* cDNA of the respective genes *- pptA* from *P. chrysogenum* NN P8 (kindly donated by Novo Nordisk) and *sfp* from *Bacillus subtilis* DSM3256 - was generated by means of RT-PCR. *PptA* cDNA was amplified using primers pptA-F (5'-TCACTATAGGGCCCGGGCGTCGACATGGTAGACCCAAGTGTGTCTGGA-3') and pptA-R (5'-TAGCTAGCCGCGGTACCAAGCTTATTAAAAGCTTGGTAAATCCCGTAG-3'), while primers sfp-F (5'-CCGTAATACGACTCACTATAGGGCATGAAGATTTACGGAATTTATATG-3') and sfp-R (5'-TAGCTAGCCGCGGTACCAAGCTTATTATAAAAGCTCTTCGTACGAG-3') were used for amplification of *sfp* cDNA. Both primer pairs contain tails, which are homologous to the vector sequences flanking *npgA* in pESC-npgA-pcbAB. *NpgA* in this vector was replaced with *pptA* and sfp, respectively, by linearising pESC-npgA-pcbAB with Smal and transforming it into CEN.PK113-9D (obtained from P. Kötter, University of Frankfurt, Germany) together with the respective cDNA fragment. Hereby, plasmids pESC-pptA-pcbAB (Fig. 2) and pESC-sfp-pcbAB (Fig. 3) were formed inside the transformed yeast cells by homologous recombination. The plasmids were isolated from the resulting transformants and confirmed by sequencing.
It had been shown previously, that changing the nucleotide composition of the 5'end towards the yeast codon bias can enhance the expression of heterologous proteins in S. *cerevisiae* (Batard et al. 2000; Mutka et al. 2006). Therefore, primers pcbAB-F (5'-AACCCTCACTAAAGGGCGGCCGCAATGACTCAATTGAAACCACCAAATG GTACTACTCCAATTGGTTTTTCTGCTACTACATCTTTGAATGCTAGTGGT AGTTCTAGTGTGAAAAATGGG-3') and module1-R (5'-GTCGCATTTCTCCTGCTGCTTGTT-3') were used to amplify the 5' region of *pcbAB,* thereby optimising the first 27 codons of the gene with regard to the yeast codon bias. Plasmid pESC-npgA-pcbAB was cut with *Not*I/*EcoR*I in order to eliminate the original 5' region. Vector and PCR fragment were co-transformed into CEN.PK113-9D to generate pESC-optpcbAB by homologous recombination as described above. The sequence of the new 5'region was confirmed by sequencing.
All four plasmid constructs were transformed into CEN.PK113-9D and ACV formation by the resulting transformants was proven by LC-MS analysis

### Example 2. LC-MS analysis of ACV formation

Yeast strains expressing ACVS and a PPTase were grown in shake flasks containing galactose as carbon source. Cells were harvested by centrifugation and re-dissolved in either methanol or acetonitrile. Mechanical disruption of the cells was achieved using glass-beads. After centrifugation, the supernatant was run at 20°C on HPLC (Agilent 1100)-MS (Micromass LCT) with Phenomenex Gemini 3u C6-Phenyl 110A 50x2 mm column (with 2 mm guard column). A linear gradient of H₂O (containing 20 mM formic acid) and methanol (containing 20 mM formic acid) was used. The gradient was changed from 0 % methanol to 100 % over 20 min and then maintained at 100 % methanol for 5 min before returning to starting conditions. The flow rate was 0.3 ml/min. Mass spectra were collected from m/z 100 to 900.

### Example 3. Integration of pcbAB and npgA into the yeast genome

In order to obtain a stable, ACV producing strain, the two genes *pcbAB* and *npgA* were integrated into the yeast genome using a tri-partite strategy (Fig. 4). As an integration site, the long terminal repeat (LTR) YCRWdelta11 was chosen. The 5'region of the integration site was PCR amplified using primers P4 (5'-GTTGCCGCAAACCAAAAA-3') and Pxc024 (5'-CTCGAAGATCCAGCTGCATTTGTTGGGATTCCATTGTTGA-3'). Primers Pxc016 (5'-AATGCAGCTGGATCTTCGAG-3') and Pxc017 (5'-GTAACCTGGCCCCACAAACC-3') were used to amplify the CYC1 terminator, *npgA,* and 0.3 kb of the GAL1 promoter from vector pESC-npgA-pcbAB. Since primer Pxc024 contained a tail complementary to Pxc016, both PCR fragments could be combined in a fusion PCR using both fragments together as template and the outer primers P4 and Pxc017 for amplification. The resulting PCR product, here referred to as fragment 1, was cloned into vector pCR-BluntII-TOPO (invitrogen) for sequence verification. Fragment 2 containing the bi-directional GAL1/GAL10 promoter and the *pcbAB* gene was isolated from vector pESC-npgA-pcbAB after restriction with *Xma*I/*Pac*I*.* An additional fragment (fragment 3) was generated by fusion PCR from three PCR products: the 3'end of *pcbAB* and the ADH1 terminator was amplified from pESC-npgA-pcbAB using primers Pxc018 (5'-CTCAACGGACTGGACAGCTT-3') and Pxc019 (5'-CTACCCAGAATCACGATCCCATTACGCCAGCTGAATTGGA-3'), the *Kluyveromyces lactis* URA3 locus flanked by direct repeats was amplified from vector pWJ1042 (Reid et al. 2002) using primers Pxc20 (5'-GGGATCGTGATTCTGGGTAG-3') and Pxc21 (5'-TGTGATTATCGTTGGGTCCAGTTTTCCCAGTCACGACGTT-3'), and the 3' region of the integration site was amplified with the aid of primers Pxc22 (5'-TGGACCCAACGATAATCACA-3') and Pxc23 (5'-TTCATTGGTCCTGTTGATGG-3'). Primer Pxc19 contained a tail complementary to Pcx20 and the tail of primer Pxc21 was complementary to Pxc22, which allowed the fusion of the three fragments in a PCR reaction using the outer primers (Pxc18 and Pxc23) for amplification. Fragment 3 was cloned into vector pCR-BluntII-TOPO and its sequence was verified. Fragment 1 and 3 were separated from the cloning vector by restriction with *Nsil* and transformed into CEN.PK113-11C (obtained from P. Kötter, University of Frankfurt, Germany) simultaneously with fragment 2. Fragment 1+2 and fragment 2+3 share 306 bp and 151 bp overlaps, respectively. This allowed the combination of all three fragments by homologous recombination, before integration into the yeast genome at the desired integration site. Correct integration was confirmed by PCR and sequencing of the resulting PCR products. Subsequently, the cells were cultivated on plates containing 5-flouroorotic acid (5-FOA). This led to the selection of clones that had lost the *K.I. URA3* marker via homologous recombination between the two flanking direct repeats.
ACV production of a transformant was proven by LC-MS analysis.

### Example 4. Plasmid based expression of pcbC, penDE, and phI in S. cerevisiae

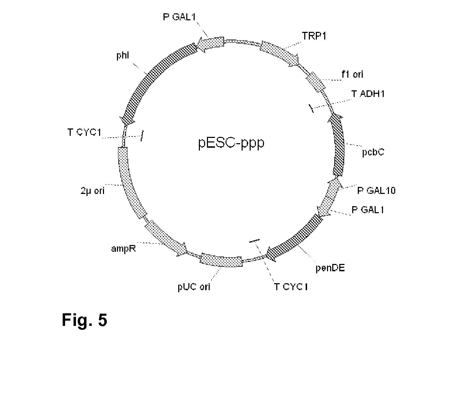

CDNA of *penDE* encoding isopenicillin N acyltransferase was amplified by means of RT-PCR from *P. chrysogenum* Wis54-1255 (ATCC 28089) RNA using primers penDE-1 (5'-CCGGATCCGATGCTTCACATCCTCTGTCAA-3'; containing a *Bam*HI site indicated in bold type) and penDE-2 (5'-TTA**GCTAGC**TCAAAGCCTGGCGTTGAG-3'; containing a *Nhel* site indicated in bold type), cut with *Bam*HI/*Nhe*I and cloned into the pESC-TRP vector (Stratagene) restricted with the same enzymes. This led to the formation of plasmid pESC-penDE. The isopenicillin N synthetase encoding gene *pcbC* was PCR amplified from cosmid pCX3.2 (see above) using primers pcbC-1 (5'-GTATCGATGGCTTCCACCCCCAAG-3'; a *Cla*I restriction site is indicated in bold type) and pcbC-2 (5'-C**TTAATTAA**TCATGTCTGGCCGTTCTTGT; a *Pac*I restriction site is indicated in bold type). The resulting fragment and plasmid pESC-penDE were cut with *Cla*I/*Pac*I and ligated to generate plasmid pESC-penDE-pcbC. In a RT-PCR approach, RNA of Wis54-1255 was used to synthesize cDNA of *phl* encoding phenylacetyl-CoA ligase with the aid of primers phl-1 (5'-CC**GGATCC**GATGGTTTTTTTACCTCCAAAGG-3'; a *Bam*HI restriction site is indicated in bold type) and phl-2 (5'-TTA**GCTAGC**TTAGATCTTGCTACCAGCCTTT-3'; containing a *Nhel* site indicated in bold type). The fragment was cut with *Bam*HI/*Nhe*I and cloned into pESC-TRP restricted with the respective enzymes. In order to express *phl* from the same vector as *penDE* and *pcbc,* its cDNA was amplified after cloning into pESC-TRP together with the GAL1 promoter and the CYC1 terminator using primers phl-GAL (5'-GAAGGATGAGACTAATCCAATTGAACGGATTAGAAGCCGCCGA-3') and phl-CYC (5'-GCTGTTCTATATGCTGCCACTCCTCTTCGAGCGTCCCAAAACCT-3').
Both primers contain tails with homology to the sequences flanking the unique *Xcm*I restriction site situated between the 2µ origin of replication and the TRP1 marker gene in pESC-penDE-pcbC. Therefore, it was possible to generate plasmid pESC-ppp (Fig. 5) by transformation of yeast with *Xcm*I restricted pESC-penDE-pcbC together with the PCR fragment through homologous recombination. Vector pESC-ppp was isolated from the transformed yeast cells and the sequences of the three genes were verified.

### Example 5. Domain shuffling of the first ACVS module.

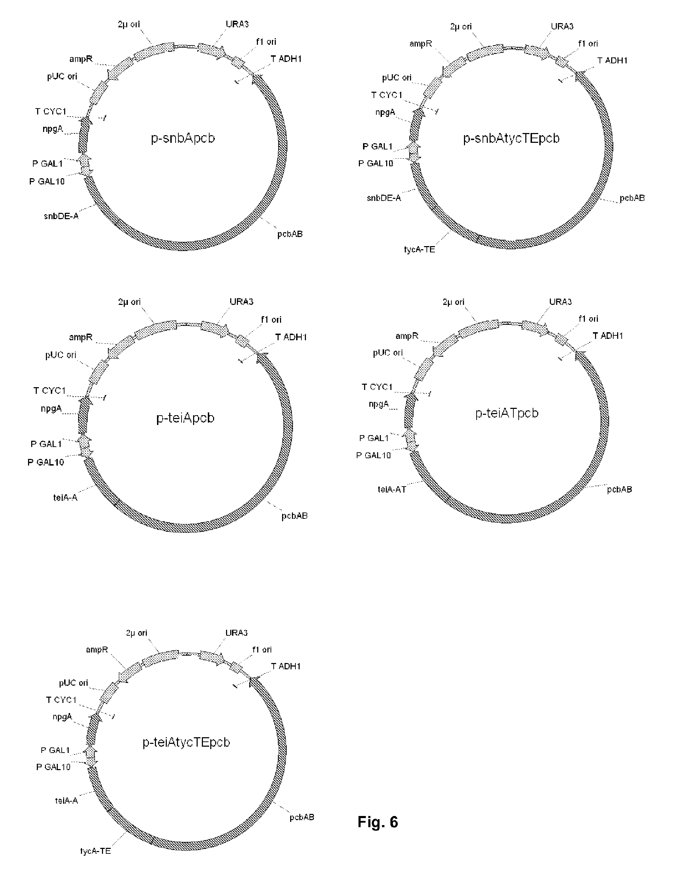

The first ACVS module was either partially or entirely exchanged against domains from different non-ribosomal peptide synthetases. For this purpose, gene fragments encoding adenylation (A), thiolation (T) and/or epimerization (E) domains were PCR amplified from the *teiA* gene of *Actinoplanes teichomyceticus* ATCC 31121, the *snbDE* gene of *Streptomyces pristinaespiralis* ATCC 25486, and the *tycA* gene of *Bacillus brevis* ATCC 8185 (the two letter strains were kindly donated by M. Marahiel, University of Marburg) as follows using genomic DNA of the respective bacterial strains as template: The gene fragment encoding the L-phenylglycine activating A domain of *snbDE* was amplified using primer snb-A-F (5'-AACCCTCACTAAAGGGCGGCCGCAATGAGAGGTAGAAGAACTGCTGAT GCTTGTTTGCCTAGAAGAATTGCCGAACAGGCCGCCCG-3') as a forward and either snb-A-R1 (5'-GCCTCGAAGGTCGGAACGGACCTCGGCGCCGCCGCCCC-3') or snb-A-R3 (5'- GAGGATGGTAGGCAGCCTGGCTGGCGCCGCCGCCCCA-3') as a reverse primer. Accordingly, the *teiA* gene section coding for the hydroxyphenylglycine activating A domain was amplified using tei-A-F (5'-AACCCTCACTAAAGGGCGGCCGCAATGAATTCTGCAGCTCAAGCAACAT CTACTGTTCCAGAATTGTTAGCCAGGCAGGTGACCAGGGCC-3') as forward and either tei-A-R1 (5'-GCCTCGAAGGTCGGAACGGACCTCAGCGTCCGCCGCGAAGAC-3') or tei-A-R3 (5'-GAGGATGGTAGGCAGCCTGGCTAGCGTCCGCCGCGAAGAC-3') as reverse primer. For amplification of the respective A and T domain encoding gene segment tei-A-F (see above) and teiA-T-R (5'-GTCGCATTTCTCCTGCTGCTTGTTAGCGCCCGCCACGGCC-3') were used as primers. Finally, the T and E domain encoding fragment of *tycA* was amplified with the aid of primers tyc-T-F (5'-AGCCAGGCTGCCTACCATCCTC-3') and tyc-E-R (5'-GTCGCATTTCTCCTGCTGCTTGTTGCGCAGTGTATTTGCAAGCAATTC-3'). As *snbDE* and *teiA* originate from organisms with a very high GC content, the nucleotide composition of the first 15 codons of their amplified gene sections was optimised towards the yeast codon bias (see sequences of primers snb-A-F and tei-A-F). Primers snb-A-F and tei-A-F contain tails homologous to the 3'end of the GAL10 promoter, while the tails of primers snb-A-R1, tei-A-R1, tei-T-R, and tyc-E-R are homologous to sequences within the *pcbAB* gene. Furthermore, the tails of primers snb-A-R3 and tei-A-R3 represent the reverse complement sequences of primer tycA-T-F. This allowed the construction of the following plasmids by homologous recombination in yeast using the respective PCR products and pESC-npgA-pcbAB, which had been cut by *Not*I/*Eco*NI, thus eliminating the 5' part of *pcbAB:* In p-snbApcb, the first A domain encoding region *of pcbAB* has been replaced by the A domain encoding fragment of *snbDE,* p-snbAtycTEpcb contains the A domain encoding section of *snbDE* and the T and E domain encoding part of *tycA,* p-teiApcb includes the A domain encoding section of *teiA,* p-teiATpcb holds the A and T domain encoding region of *teiA,* and p-teiAtycTEpcb contains the A domain encoding fragment of *teiA* and the T and E domain encoding part of *tycA* (Fig 6). All fragments generated by PCR amplification were verified by sequencing.

### Example 6. Expression of NRPS modules on different vectors

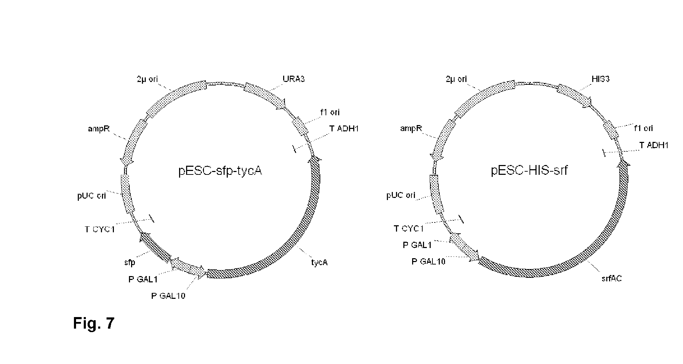

Modules of NRPSs that are located on different polypeptide chains can interact via short communication-mediating (COM) domains (Hahn and Stachelhaus, 2004). In order to test whether this system also works in yeast, two plasmids were constructed. The *tycA* gene of *Bacillus brevis* ATCC 8185 was PCR amplified using primers tycA-F (5'-GAATTCAACCCTCACTAAAGGGCGGCCGCATGTTAGCAAATCAGGCCAA T-3') and tycA-R1 (5'-CCAAACCTCTGGCGAAGAATTGTTAATTAATTAGCGCAGTGTATTTGCAA G-3'). Plasmid pESC-sfp-pcbAB was cut with *Not*I/*Pac*I to remove the *pcbAB* gene. Since primers tycA-F and tycA-R1 contain tails homologous to the respective ends of the so linearised vector, plasmid pESC-sfp-tycA (Fig. 7) was generated by homologous recombination in yeast after co-transformation of the vector together with the PCR fragment into CEN.PK 113-11C. Accordingly, plasmid pESC-HIS-srf (Fig. 7) was generated using *Not*I/*Pac*I linearised pESC-HIS vector (Stratagene) and the *srfAC* gene of *Bacillus subtilis* DSM3256 amplified with the aid of primers srfAC-F (5'-GAATTCAACCCTCACTAAAGGGCGGCCGCATGAGTGTTTTTAGCAAGGA ACAGGTTCAAGATATGTATGCTCTAACTCCGATGCAG-3') and srfAC-R1 (5'-CCAAACCTCTGGCGAAGAATTGTTAATTAATTATGAAACCGTTACGGTTT GT-3'). Hereby, the sequence encoding the N-terminal COM domain of SrfAC was changed so that it encodes the N-terminal COM domain of TycB, which mediates interaction with TycA. This was achieved by introducing the appropriate nucleotide changes into primer srfAC-F.

### References

Batard, Y., Hehn, A., Nedelkina, S., Schalk, M., Pallett, K., Schaller, H., and Werck-Reichhart, D. 2000. Increasing expression of P450 and P450-reductase proteins from monocots in heterologous systems. Arch. Biochem. Biophys. 379:161-169.
Hahn, M., and Stachelhaus, T. 2004. Selective interaction between nonribosomal peptide synthetases is facilitated by short communication-mediating domains. Proc. Natl. Acad. Sci. 101:15585-15590.
Mutka, S.C., Bondi, S.M., Carney, J.R., Da Silva, N.A., and Kealey, J.T. 2006. Metabolic pathway engineering for complex polyketide biosynthesis in Saccharomyces cerevisiae. FEMS Yeast Res 6:40-47.
Reid, R.J.D., Sunjevaric, I., Kedacche, M., and Rothstein, R. 2002. Efficient PCR-based gene disruption in Saccharomyces strains using intergenic primers. Yeast 19:319-328.
Smith, D.J., Burnham, M.K.R., Bull, J.H., Hodgson, J.E., Ward, J.M., Browne, P., Brown, J., Barton, B., Earl, A.J., and Turner, G. 1990. β-Lactam antibiotic biosynthetic genes have been conserved in clusters in prokaryotes and eukaryotes. EMBO J. 9:741-747.

## Claims

1. An isolated *Saccharomyces cerevisiae* comprising at least one heterologous gene encoding a non-ribosomal protein synthetase (NRPS) catalyzing the synthesis of at least one non-ribosomal peptide.

2. The isolated *Saccharomyces cerevisiae* according to any of the preceding claims, which comprises a heterologous gene encoding 4'-phosphopantetheinyl transferase.

3. The isolated *Saccharomyces cerevisiae* according to claim 2, wherein said heterologous gene encoding 4'-phosphopantetheinyl transferase originates from a microorganism, such as from a fungal microorganism.

4. The isolated *Saccharomyces cerevisiae* according to claim 3, wherein said heterologous gene encoding 4'-phosphopantetheinyl transferase originates from *Bacillus subtilis, Aspergillus nidulans* or from *Penicillium chrysogenum.*

5. The isolated *Saccharomyces cerevisiae* according to any of the preceding claims, wherein said at least one heterologous gene encoding a NRPS encodes a single polypeptide or several associated polypeptides.

6. The isolated *Saccharomyces cerevisiae* according to any of the preceding claims, wherein said at least one heterologous gene encoding a NRPS comprises a gene encoding ACV synthetase (ACVS).

7. The isolated *Saccharomyces cerevisiae* according to any of the preceding claims, which comprises a gene encoding isopenicillin synthetase (IPNS).

8. The isolated *Saccharomyces cerevisiae* according to any of the preceding claims, which comprises a gene encoding phenylacetyl-CoA ligase and a gene encoding an acyltransferase.

9. The isolated *Saccharomyces cerevisiae* according to any of claims 6-8, which comprises a gene encoding acyltransferase and a gene encoding expandase.

10. The isolated *Saccharomyces cerevisiae* according to any of the preceding claims, wherein NRPS modules have been shuffled so as to enable said *Saccharomyces cerevisiae* to produce δ-(L-α-aminoadipyl)-L-cysteinyl-D-valine (ACV) derivatives.

11. Process for the manufacture of a non-ribosomal peptide comprising the steps of :
a) cultivating an isolated *Saccharomyces cerevisiae* according to any of claims 1-10 in a suitable growth medium,
b) optionally feeding of one or more precursors for said non-ribosomal peptide during said cultivation,
c) harvesting the spent cultivation medium,
d) isolating the non-ribosomal peptide from said spent cultivation medium.

12. Process for the manufacture of ampicillin, amoxicillin, cephalexin or cefadroxil comprising the steps of :
a) cultivating an isolated *Saccharomyces cerevisiae* according to any of claims 1-10 in a suitable growth medium,
b) feeding of phenylglycine or p-hydroxyphenylglycine during said cultivation,
c) harvesting spent cultivation medium,
d) isolating the ampicillin, amoxicillin, cephalexin or cefadroxil from said spent cultivation medium.

13. Use of *Saccharomyces cerevisiae* as host cell for the industrial manufacture of a non-ribosomal peptide.

14. Use of *Saccharomyces cerevisiae* as host cell for the synthesis of a non-ribosomal peptide.

15. The use according to any of claims 13-14, wherein said *Saccharomyces cerevisiae* comprises at least one heterologous gene encoding a NRPS.

16. The use according to claim 15, wherein said *Saccharomyces cerevisiae* comprises a heterologous gene encoding 4'-phosphopantetheinyl transferase.

17. The use according to any of claims 13-16, wherein NRPS modules in said *Saccharomyces cerevisiae* have been shuffled such that at least one ACV derivative is produced.

18. The use according to claim 17, wherein said at least one ACV derivative is selected from D-HPG-L-Cys-D-Val, D-PG-L-Cys-D-Val or a mixture thereof.

19. Use of an isolated *Saccharomyces cerevisiae* according to any of claims 1-10 for the manufacture of a compound selected from the group consisting of 6-APA, 7-ADCA, ampicillin, cephalexin, amoxocillin or cefadroxil.

20. Use of an isolated *Saccharomyces* according to any of claims 1-10, for the generation of a library of antibiotics.

21. Use of an isolated *Saccharomyces* according to any of claims 1-10, for the manufacture of a NRPS.
